(19) Europäisches Patentamt
European Patent Office
Office européen des brevets

(11) EP 1 645 579 A1

(12) DEMANDE DE BREVET EUROPEEN

(43) Date de publication:
**12.04.2006 Bulletin 2006/15**

(51) Int Cl.:
***C08G 18/08** (2006.01)* ***A61K 8/30** (2006.01)*
***A61K 8/06** (2006.01)*

(21) Numéro de dépôt: **05300762.1**

(22) Date de dépôt: **20.09.2005**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **21.09.2004 FR 0452109**

(71) Demandeur: **L'OREAL**
**75008 Paris (FR)**

(72) Inventeurs:
- **MOUGIN, Nathalie**
  **75011, PARIS (FR)**
- **SCHULTZE, Xavier**
  **77340, PONTAULT-COMBAULT (FR)**

(74) Mandataire: **Catherine, Alain et al**
**Cabinet Harlé & Phélip**
**7, rue de Madrid**
**75008 Paris (FR)**

# (54) Polyuréthane cationique ou cationisable à caractère élastique

(57) La présente invention a pour objet un polyuréthane cationique ou cationisable à caractère élastique formé de la réaction :

(a1) d' au moins un motif cationique ou cationisable résultant d'au moins une amine tertiaire ou quaternaire présentant au moins deux fonctions réactives à H labiles
(a2) d'au moins un mélange d'au moins deux motifs non ioniques différents présentant au moins deux fonctions réactives à H labiles
(b) d'au moins un composé comportant au moins deux fonctions isocyanate,

le mélange de motifs non ioniques (a2) est constitué d'un mélange d'au moins un premier polymère non ionique choisi parmi les homopolymères et copolymères d'oléfines, et d'au moins un second polymère non ionique différent d'un copolymère d'oléfine et choisi parmi les homopolymères et copolymères non ioniques, le ou lesdits premiers polymères non ioniques et le ou lesdits seconds polymères non ioniques portant à leurs extrémités des fonctions réactives à hydrogène labile et ayant une température de transition vitreuse (Tg), mesurée par analyse enthalpique différentielle, inférieure à 10 °C.

EP 1 645 579 A1

## Description

La présente invention concerne un nouveau polyuréthane cationique ou cationisable à caractère élastique ainsi que son utilisation dans des compositions cosmétiques.

La formation de dépôts et de films à propriétés élastiques fait depuis toujours l'objet d'importantes recherches en cosmétique. En effet, la plupart des zones du corps humain susceptibles de recevoir des dépôts cosmétiques, telles que la peau, les lèvres, les cheveux, les cils et les ongles sont soumises à des déformations et contraintes mécaniques importantes. Les films et dépôts cosmétiques doivent pouvoir résister à ces contraintes et suivre ces déformations sans se casser.

L'utilisation de polyuréthanes en cosmétique est connue depuis longtemps et est décrite par exemple dans les brevets WO 94/13724 et EP 0 619 111.

Les polyuréthanes divulgués dans ces documents présentent cependant des températures de transition vitreuse (Tg) supérieures à la température ambiante (20 °C), c'est-à-dire qu'à la température ambiante ils sont à l'état vitreux et forment des films cassants inacceptables pour une application cosmétique.

Il existe certes des polymères physiologiquement acceptables présentant des températures de transition vitreuse basses, comme par exemple les polymères acryliques, mais ces polymères forment généralement des dépôts très collants, ce qui présente un inconvénient dans la plupart des applications cosmétiques.

On connaît par ailleurs de la demande WO 02/32 978 des polyuréthanes physiologiquement acceptables formant des films non collants, non cassants et capables de déformations plastiques et élastiques. Ces propriétés viscoélastiques intéressantes sont dues à la présence, dans le polymère, de longs motifs macromoléculaires ayant une température de transition vitreuse relativement faible et qui de ce fait, à température ambiante, ne se trouvent pas à l'état vitreux.

Le but de la présente invention est de fournir des polyuréthanes physiologiquement acceptables présentant des propriétés filmogènes et viscoélastiques améliorées.

La présente invention a par conséquent pour objet un polyuréthane cationique ou cationisable à caractère élastique, comprenant au moins un motif cationique ou cationisable, et au moins deux motifs non ioniques.

Plus particulièrement, l'invention a pour objet un polyuréthane cationique ou cationisable à caractère élastique formé de la réaction :

(a1) d' au moins un motif cationique ou cationisable résultant d'au moins une amine tertiaire ou quaternaire présentant au moins deux fonctions réactives à H labiles,
(a2) d'au moins un mélange d'au moins deux motifs non ioniques différents présentant au moins deux fonctions réactives à H labiles,
(b) d'au moins un composé comportant au moins deux fonctions isocyanate,
le mélange de motifs non ioniques (a2) étant constitué d'un mélange d'au moins un premier polymère non ionique choisi parmi les homopolymères et copolymères d'oléfines, et d'au moins un second polymère non ionique différent d'un copolymère d'oléfine et choisi parmi les homopolymères et copolymères non ioniques, le ou lesdits premiers polymères non ioniques et le ou lesdits seconds polymères non ioniques portant à leurs extrémités des fonctions réactives à hydrogène labile et ayant une température de transition vitreuse (Tg), mesurée par analyse enthalpique différentielle, inférieure à 10 °C.

Par motif cationique ou cationisable, on entend au sens de la présente invention tout motif qui, soit par sa nature chimique propre, soit en fonction du milieu et/ou du pH dans lequel il se trouve, sera sous forme cationique.

L'invention a également pour objet l'utilisation d'un polyuréthane cationique ou cationisable à caractère élastique tel que défini ci-dessus dans des compositions cosmétiques en vue d'améliorer les propriétés visco-élastiques des dépôts et films cosmétiques obtenus à partir de ces compositions.

Elle a en particulier pour objet l'utilisation de ce polyuréthane dans des laques et des compositions de coiffage, dans des vernis à ongles, dans des compositions de maquillage de la peau, des lèvres et des phanères, et pour former des films protecteurs pour les ongles.

L'invention a encore pour objet l'utilisation de ce polyuréthane pour la mise en forme des cheveux et en particulier pour la formulation de laques, de mousses ou de gels de coiffage.

L'invention a également pour objet des compositions cosmétiques contenant un polyuréthane cationique ou cationisable à caractère élastique tel que défini ci-dessus.

Le polyuréthane à caractère élastique selon l'invention, grâce à son caractère cationisable, présente l'avantage d'avoir une excellente affinité pour les substrats kératiniques tels que les cheveux, les ongles et la couche cornée de l'épiderme auxquels la kératine confère une charge négative.

L'utilisation du polyuréthane cationique ou cationisable à caractère élastique selon l'invention dans des laques et des compositions de coiffage permet d'améliorer la souplesse de la coiffure, c'est-à-dire d'obtenir une tenue des

cheveux plus naturelle et plus durable que celle obtenue avec des polymères fixants de l'art antérieur.

On peut utiliser le polyuréthane selon l'invention pour couvrir les ongles avec un film protecteur brillant résistant aux agressions mécaniques. Son incorporation dans un vernis à ongles améliore la résistance aux chocs de ceux-ci et retarde l'écaillage.

Le polyuréthane cationique ou cationisable selon l'invention peut également être utilisé pour améliorer la tenue de compositions de maquillage de la peau, des lèvres et des phanères. En effet, les produits de maquillage contenant ce polyuréthane adhèrent bien à la peau et aux phanères et les dépôts obtenus suivent les déformations des substrats kératiniques et ne tirent pas la peau.

Dans toutes ces applications, on obtient des produits non collants.

De plus, les compositions comprenant un polyuréthane selon l'invention présentent une meilleure tenue à l'humidité grâce à l'utilisation de copolymères d'oléfines qui présentent un caractère hydrophobe.

Par matière élastique, on entend au sens de la présente invention une matière macromoléculaire qui retourne rapidement à sa forme et à ses dimensions initiales après cessation d'une contrainte faible ayant produit une déformation importante.

Comme indiqué ci-dessus, le polyuréthane cationique ou cationisable à caractère élastique de la présente invention comprend au moins un motif cationique ou cationisable, et au moins deux motifs non ioniques,

La présente invention a pour objet un polyuréthane cationique ou cationisable à caractère élastique formé de la réaction :

(a1) d' au moins un motif cationique ou cationisable résultant d'au moins une amine tertiaire ou quaternaire présentant au moins deux fonctions réactives à H labiles,

(a2) d'au moins un mélange d'au moins deux motifs non ioniques différents présentant au moins deux fonctions réactives à H labiles,

(b) d'au moins un composé comportant au moins deux fonctions isocyanate,

le mélange de motifs non ioniques (a2) étant constitué d'un mélange d'au moins un premier polymère non ionique choisi parmi les homopolymères et copolymères d'oléfines, et d'au moins un second polymère non ionique différent d'un homopolymère ou copolymère d'oléfine et choisi parmi les homopolymères et copolymères non ioniques, le ou lesdits premiers polymères non ioniques et le ou lesdits seconds polymères non ioniques portant à leurs extrémités des fonctions réactives à hydrogène labile et ayant une température de transition vitreuse (Tg), mesurée par analyse enthalpique différentielle, inférieure à 10 °C.

On entend par fonctions réactives à hydrogène labile des fonctions capables, après départ d'un atome d'hydrogène, de former des liaisons covalentes avec les fonctions isocyanate des composés comportant au moins deux fonctions isocyanate. On peut citer à titre d'exemple de telles fonctions les groupes hydroxyle, amine primaire ou amine secondaire, ou encore les groupes thiol.

La polycondensation de composés portant ces fonctions réactives à hydrogène labile avec des composés comportant au moins deux fonctions isocyanate donne, selon la nature des fonctions réactives portant l'hydrogène labile (-OH, $-NH_2$, -NHR ou -SH), respectivement des polyuréthanes, des polyurées ou des polythiouréthanes. Ainsi, les polymères utilisés dans l'invention peuvent être des copoly uréthane/urée et/ou thiouréthane.

Tous ces polymères sont regroupés dans la présente demande, par souci de simplification, sous le terme de polyuréthanes.

Selon un mode de réalisation particulièrement avantageux, le ou lesdits premiers copolymères d'oléfines non ioniques et le ou lesdits seconds polymères différents d'un copolymère d'oléfines non ioniques formant les motifs non ioniques (a2) présentent une température de transition vitreuse, mesurée par analyse enthalpique différentielle, inférieure à 0 °C, de préférence inférieure à -10 °C.

De préférence, les homopolymères et copolymères d'oléfines sont des homopolymères et copolymères présentant des motifs choisis parmi les motifs d'éthylène, de propylène, de 1-butylène (ou 1-butène), de 2-butylène (ou 2-butène), d'isobutylène, de 1,2-butadiène, de 1,4-butadiène et d'isoprène.

Lorsque les polymères d'oléfines résultent de la polymérisation du butadiène, il peut s'agir de copolymères (1,2-butadiène, 1,4-butadiène).

Les homopolymères ou copolymères d'oléfines résultant de la polymérisation du butadiène peuvent subir, ultérieurement à la polymérisation, une hydrogénation des doubles liaisons résiduelles. Cette hydrogénation peut être totale ou partielle.

Dans le cas d'une hydrogénation partielle, il reste alors des teneurs faibles en doubles liaisons résiduelles. Ces polymères sont toutefois appelés copolymères d'éthylène et de butylène, par souci de simplification.

Un copolymère d'oléfine particulièrement préféré comme premier polymère non ionique est le copolymère d'éthylène et de butylène. On peut citer notamment les KRATON L, et en particulier le KRATON L2203 commercialisé par la société KRATON, et les GI3000 commercialisés par la société NISSO CHEMICAL.

On peut encore citer à titre de copolymère de polyoléfines les copolymères statistiques (1,2-butadiène / 1,4-butadiène).

Outre le ou les premiers polymères non ioniques choisis parmi les homopolymères et copolymères d'oléfines, les motifs (a2) sont choisis également parmi les homopolymères et copolymères non ioniques différents d'un polymère d'oléfines,

Les homopolymères et/ou copolymères différents d'un polymère d'oléfine formant les motifs non ioniques (a2) sont généralement choisis parmi les poly(méth)acrylates, les polyamides, les polyéthers hydrocarbonés, fluorés ou perfluorés, les polyesters, les polyalkylsiloxanes, et les polycarbonates hydrogénés, fluorés ou perfluorés.

En particulier, les polyéthers peuvent être choisis parmi les poly(oxyde d'alkylène en $C_2$-$C_4$) de masse moléculaire en nombre comprise entre 400 et 50000, de préférence le poly(oxyde de tétraméthylène). Parmi les poly(oxyde de tétraméthylène), on peut citer en particulier les polymères vendus sous la dénomination TERATHANE par la société DUPONT DE NEMOURS. On préfère en particulier les poly(oxyde de tétraméthylène) dont la masse moléculaire en nombre est égale à 1400 ou 2900.

Selon un mode de réalisation préféré, le mélange de motifs (a2) est un mélange de poly(oxyde de tétraméthylène) et de copolymère présentant des motifs d'éthylène, de 1,2-butadiène et/ou de 1,4-butadiène.

Généralement le ou les premiers polymères d'oléfines non ioniques et le ou les seconds polymères non oléfines formant les motifs non ioniques (a2) ont une masse moléculaire en nombre comprise entre 400 et 50000, de préférence entre 400 et 30000, mieux entre 400 et 25000.

En outre du ou des motifs non ioniques (a2), le polyuréthane cationique ou cationisable selon l'invention est formé d'au moins un motif cationique ou cationisable (a1) résultant d'au moins une amine tertiaire ou quaternaire présentant au moins deux fonctions réactives à hydrogène labile.

Lorsque la ou les amines tertiaires ou quaternaires formant les motifs (a1) portent plus de deux fonctions à hydrogène labile, le polyuréthane obtenu présente une structure ramifiée.

Selon un mode de réalisation préféré, la ou les amines tertiaires ou quaternaires formant les motifs cationiques ou cationisables (a1) ne présentent que deux fonctions réactives à hydrogène labile et les polyuréthanes obtenus par polycondensation ont par conséquent une structure essentiellement linéaire.

Il est bien entendu également possible d'utiliser un mélange d'amines difonctionnelles contenant une faible proportion d'amines portant plus de deux fonctions réactives à hydrogène labile.

La ou les amines tertiaires ou quaternaires formant les motifs cationiques ou cationisables (a1) sont de préférence choisies parmi les composés correspondant à l'une des formules suivantes :

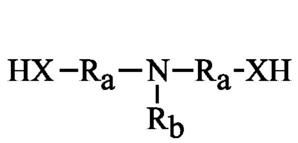

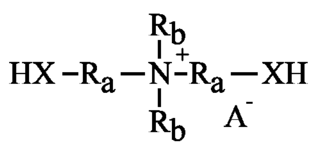

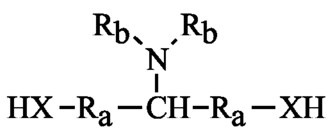

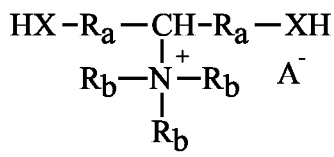

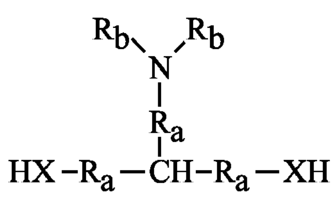

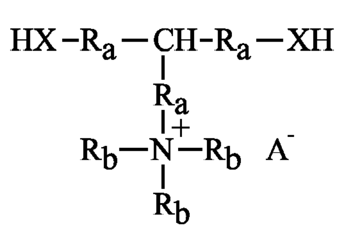

dans lesquelles

chaque $R_a$ représente indépendamment un groupe alkylène en $C_1$-$C_6$, linéaire ou ramifié, cycloalkylène en $C_3$-$C_6$

ou arylène, ou leurs mélanges ; tous pouvant être substitués par un ou plusieurs atomes d'halogène et comporter un ou plusieurs hétéroatomes choisis parmi O, N, P et S,

chaque $R_b$ représente indépendamment un groupe alkyle en $C_1$-$C_6$, cycloalkyle en $C_3$-$C_6$ ou aryle, ou leurs mélanges ; tous pouvant être substitués par un ou plusieurs atomes d'halogène et comporter un ou plusieurs hétéroatomes choisis parmi O, N, P et S,

chaque X représente indépendamment un atome d'oxygène ou de soufre ou un groupe NH ou $NR_c$, où $R_c$ représente un groupe alkyle en $C_{1-}C_6$, et

$A^-$ représente un contre-ion physiologiquement acceptable.

On peut citer à titre d'amines tertiaires particulièrement préférées pour l'obtention du polyuréthane cationique ou cationisable à caractère élastique de la présente invention la N-méthyldiéthanolamine et la N-tert-butyldiéthanolamine.
La ou les amines tertiaires et quaternaires formant les motifs cationiques ou cationisables (a1) du polyuréthane de la présente invention peuvent également être des polymères à fonction(s) amine tertiaire et/ou quaternaire, portant à leurs extrémités des fonctions réactives à hydrogène labile choisies parmi -OH, $-NH_2$, $-NHR_c$ ou -SH, où $R_c$ représente un groupe alkyle en $C_1$-$C_6$. La masse molaire moyenne en poids de ces polymères à fonctions amine tertiaire et/ou quaternaire est de préférence comprise entre 400 et 10 000.
On peut citer à titre d'exemples de tels polymères à fonction(s) amine appropriés les polyesters issus de la polycondensation de la N-méthyldiéthanolamine et de l'acide adipique.
Lorsque les amines formant les motifs cationiques ou cationisables (a1) sont des composés à fonction(s) amine tertiaire, une partie ou la totalité de ces fonctions amine doit être neutralisée par un agent de neutralisation approprié choisi parmi les acides organiques ou minéraux physiologiquement acceptables. On peut citer à titre d'exemple d'acides préférés l'acide chlorhydrique, l'acide acétique et l'acide tartrique.
Comme expliqué précédemment, le polyuréthane selon l'invention est formé par la réaction des composés (a1) et (a2) avec au moins un composé (b) comportant au moins deux fonctions isocyanate.
De préférence, le ou les composés comportant au moins deux fonctions isocyanate sont choisis parmi les diisocyanates ou les mélanges d'un diisocyanate et d'un isocyanate comportant plus de deux fonctions isocyanates, ledit isocyanate représentant de 0,1 à 10% en poids du poids dudit mélange.
Le ou les composés comportant au moins deux fonctions isocyanate peuvent être choisis parmi les diisocyanates aliphatiques, cycliques conjugués ou non, aromatiques ou non.
Ainsi, le ou les diisocyanates préférés sont choisis de préférence parmi le méthylènediphényldiisocyanate, le méthylènecyclohexanediisocyanate, l'isophoronediisocyanate, le toluènediisocyanate, le naphtalènediisocyanate, le 1,4-butanediisocyanate et le 1,6-hexanediisocyanate.
Le polyuréthane cationique ou cationisable à caractère élastique de la présente invention peuvent contenir, en plus des motifs (a1) et (a2), au moins un motif (a3) non ionique monomère contenant au moins deux fonctions à hydrogène labile capables de réagir avec le ou lesdits composés (b) comportant deux fonctions isocyanate.
De préférence, les composés non ioniques monomères formant les motifs non ioniques (a3) sont choisis parmi les diols en $C_1$-$C_{12}$, de préférence le néopentylglycol, l'hexa(éthylèneglycol), le 1,2-éthanediol, le 1,2-propanediol et le 1,3-propanediol, et les aminoalcools en $C_1$-$C_6$, de préférence l'aminoéthanol.
La viscosité du polyuréthane cationique ou cationisable selon l'invention, mesurée à 10% dans le tétrahydrofuranne, à 25°C, avec un viscosimètre Brookfield, module aiguille, est généralement comprise entre 1 et 1000 cps, de préférence entre 1 et 100 cps, mieux entre 2 et 80 cps. Le polyuréthane est caractérisé sous forme non neutralisée.
Le paramètre physique caractérisant les propriétés visco-élastiques du polyuréthane cationique ou cationisable décrit précédemment est sa recouvrance en traction. Cette recouvrance est déterminée par essai de fluage en traction consistant à étirer rapidement une éprouvette jusqu'à un taux d'allongement prédéterminé, puis à relâcher la contrainte et à mesurer la longueur de l'éprouvette.
L'essai de fluage utilisé pour la caractérisation du polyuréthane cationique ou cationisable à caractère élastique selon l'invention se déroule de la manière suivante :

On utilise, comme éprouvette, un film du polyuréthane ayant une épaisseur de 500 ± 50 mm, découpé en bandes de 80 mm x 15 mm. Ce film de copolymère est obtenu par séchage, à une température de 22 ± 2 °C et à une humidité relative de 50 ± 5 %, d'une solution ou dispersion à 3 % en poids dudit polyuréthane dans de l'eau et/ou de l'éthanol.

Chaque bande est fixée entre deux mors, distants de 50 ± 1 mm l'un de l'autre, et est étirée à une vitesse de 20 mm/minute (dans les conditions de température et d'humidité relative ci-dessus) jusqu'à un allongement de 50 % ($\varepsilon_{max}$), c'est-à-dire jusqu'à 1,5 fois sa longueur initiale. On relâche alors la contrainte en imposant une vitesse de retour égale à la vitesse de traction, soit 20 mm/minute, et on mesure l'allongement de l'éprouvette (exprimé en % par rapport à la longueur initiale) immédiatement après retour à charge nulle ($\varepsilon_i$).

La recouvrance instantanée ($R_i$) est calculée à l'aide de la formule suivante :

$$R_i\ (\%) = ((\varepsilon_{max} - \varepsilon_i)/\ \varepsilon_{max})\ x\ 100$$

Le polyuréthane cationique ou cationisable à caractère élastique de la présente invention a de préférence une recouvrance instantanée ($R_i$), mesurée dans les conditions indiquées ci-dessus, comprise entre 5 % et 95 %, en particulier comprise entre 20 % et 90 % et idéalement entre 35 et 85 %.

La température de transition vitreuse (Tg) des polymères non ioniques formant les motifs (a2) et du polyuréthane cationique ou cationisable de la présente invention est mesurée par analyse enthalpique différentielle (DSC, *differential scanning calorimetry*) selon la norme ASTM D3418-97.

Le polyuréthane cationique ou cationisable à caractère élastique de la présente invention présente de préférence au moins deux températures de transition vitreuse, dont une au moins est inférieure à 10 °C, de préférence inférieure à 0 °C et mieux encore inférieure à -10 °C, et au moins une autre est supérieure ou égale à la température ambiante (20 °C).

La recouvrance instantanée et par conséquent les propriétés viscoélastiques du polyuréthane selon l'invention dépend des fractions des différents motifs monomères (a1), (a2), (a3) et de la quantité de composé (b) comprenant au moins deux fonctions isocyanate.

La fraction de motifs (a1) doit être suffisante pour conférer aux polymères leur charge positive responsable de leur bonne affinité pour les substrats kératiniques. Le ou les motifs (a2) doivent représenter une fraction en poids suffisante pour que les polyuréthanes présentent au moins une température de transition vitreuse inférieure à 10 °C et ne forment pas des films cassants.

De manière générale, les motifs cationiques ou cationisables (a1) représentent de 0,1 à 90 %, de préférence de 1 à 30 %, mieux de 1 à 20% en poids, les motifs non ioniques (a2) de 10 à 99,9 %, de préférence de 20 à 99 %, mieux de 30 à 85% en poids et les motifs (a3) de 0 à 50 % en poids, de préférence de 0 à 40 %, mieux de 0 à 30% en poids du polyuréthane cationique ou cationisable.

Le ou les composés (b) comprenant au moins deux fonctions isocyanate sont présents en une quantité essentiellement stoechiométrique par rapport à la somme de la ou des amines tertiaires ou quaternaires formant les motifs (a1), du ou des premiers polymères non ioniques et du ou des seconds polymères non ioniques formant les motifs (a2), et du ou des composés non ioniques monomères formant les motifs (a3).

En effet, l'obtention de polyuréthanes ayant des masses molaires importantes suppose un nombre de fonctions isocyanate pratiquement identique au nombre de fonctions à hydrogène labile. L'homme du métier saura choisir un éventuel excès molaire de l'un ou de l'autre type de fonction pour ajuster la masse molaire à la valeur désirée.

Comme indiqué précédemment, le polyuréthane cationique ou cationisable à caractère élastique selon l'invention peut être incorporé dans de nombreuses compositions cosmétiques dont il améliore les propriétés cosmétiques.

La quantité de polyuréthane présente dans les différentes compositions dépend bien entendu du type de composition et des propriétés recherchées et peut varier à l'intérieur d'une gamme très large, comprise généralement entre 0,1 et 90 % en poids, de préférence entre 1 et 50 % en poids, rapporté à la composition cosmétique finale.

Lorsque le polyuréthane cationique ou cationisable à caractère élastique est incorporé dans des laques pour cheveux, sa teneur est généralement comprise entre 0,5 et 15 % en poids. Dans des vernis à ongles, il représente généralement de 0,5 et 40 % en poids de la composition. Dans des compositions de maquillage de la peau, des lèvres et des phanères, il représente généralement de 0,5 à 20 % en poids de la composition. Dans des compositions de produit de coiffage, il représente généralement de 0,5 à 10% en poids de la composition. Dans des compositions de shampooing, il représente généralement de 0,1 à 5% en poids de la composition.

La composition selon l'invention peut comprendre en outre au moins un adjuvant cosmétiquement acceptable classiquement utilisé dans les compositions cosmétiques destinées à être appliquées sur les fibres kératiniques. On peut citer en particulier les gélifiants et/ou épaississants, tels que les épaississants polymères associatifs ou non, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les polymères fixants ou non, les agents bactéricides et les agents anti-pelliculaires.

La composition selon l'invention peut se présenter sous la forme d'une lotion, épaissie ou non, d'une crème, épaissie ou non, d'un gel, d'une mousse ou de toute autre forme appropriée. Elle peut éventuellement être conditionnée dans un flacon pompe ou dans un récipient aérosol.

On peut également envisager l'utilisation du polyuréthane cationique ou cationisable à caractère élastique

selon l'invention par exemple pour former un film protecteur sur les ongles.

Le polyuréthane cationique ou cationisable à caractère élastique selon l'invention peut notamment être utilisé en application rincée sur les cheveux.

**Exemple 1** : Synthèse d'un polyuréthane cationique ou cationisable à caractère élastique

On réalise la synthèse d'un polyuréthane cationique ou cationisable à caractère élastique selon l'invention, par réaction des composés suivants (en poids) :

a1) 8,6% de N-méthyldiéthanolamine (notée NMDEA),
a2) 8,4% d'un poly(oxyde de tétraméthylène) (noté PTMO) de masse molaire moyenne en poids égale à 1400, et 61,6% en poids d'un copolymère éthylène-butylène commercialisé sous la référence KRATON L2203 (de masse molaire moyenne en poids égale à 2000) par la société KRATON,
b) 21,4% en poids d'isophoronediisocyanate (noté IPDI).

1. Mode opératoire :

Dans un réacteur de 500 ml équipé d'un réfrigérant et d'une agitation mécanique, on place le PTMO (8,4 g), le KRATON L2203 (61,6 g) et la NMDEA (8,6 g) dans 100 g de THF (l'extrait sec est égal à 44 % en poids).

Le milieu réactionnel est homogénéisé sous agitation à 100 tour/min au reflux du THF pendant 45 minutes. La température du milieu réactionnel est de 80°C.

L'IPDI (21,4 g) et 20 g de THF sont placés dans une ampoule d'addition qui est fixée sur le réacteur. Ce mélange est ajouté au milieu réactionnel sur 45 minutes.

Après l'addition du mélange, le milieu est laissé au reflux pendant 90 minutes.

0,1 g de dibutyl dilaurate d'étain sont alors ajoutés, ce qui entraîne une augmentation du reflux de courte durée (5 min).

Le milieu réactionnel est laissé au reflux pendant 210 minutes à l'issue desquelles le pic caractéristique des groupements NCO a disparu en Infra-Rouge (2250 $cm^{-1}$). On observe une augmentation progressive de la viscosité.

On ajoute 10 g d'éthanol et on laisse encore 60 minutes au reflux afin de détruire d'éventuelles traces d'IPDI qui n'auraient pas été détectées par l'Infra-Rouge.

2. Mise en dispersion aqueuse :

A température ambiante, le milieu réactionnel est dilué au THF jusqu'à obtenir un extrait sec de 30% en poids.

72 ml d'acide chlorhydrique 1N sont ajoutés sous agitation mécanique (200 tour/minutes), ce qui permet la neutralisation à 100% des fonctions amines tertiaires. On observe une forte augmentation de la viscosité.

400g d'eau sont ensuite ajoutés au goutte à goutte à l'aide d'une ampoule d'addition, ce qui entraîne un blanchiment du milieu et une diminution de la viscosité .

Le mélange ainsi obtenu est ensuite évaporé au rotavapor à une température de bain de 50°C, en augmentant progressivement le vide de façon à éliminer le solvant.

On obtient un polyuréthane en dispersion dans l'eau. La taille de particule est égale à 160 nm. L'extrait sec est égal à 29,6% en poids. Le pH est égal à 4,65.

Les tailles de particules ont été mesurées par diffusion de la lumière à l'aide d'un granulomètre Coulter N4 SD.

L'extrait sec est mesuré en plaçant les échantillons dans une étuve ventilée à 70°C pendant une nuit.

La viscosité de la forme non neutralisée à 10% dans le THF à 25°C est de 20 cps.

L'élasticité est testée de la façon suivante : une éprouvette est réalisée à partir d'un film réalisé par séchage de la dispersion aqueuse. On obtient une recouvrance élastique instantanée après 100% de déformation de 50%.

**Exemple 2 :** Synthèse d'un polyuréthane cationique ou cationisable à caractère élastique

On réalise la synthèse d'un polyuréthane cationique ou cationisable à caractère élastique selon l'invention, par réaction des composés suivants (en poids) :

a1) 8,6% de N-méthyldiéthanolamine (notée NMDEA),
a2) 8,5% d'un poly(oxyde de tétraméthylène) (noté PTMO) de masse molaire moyenne en poids égale à 1400, et 61,6% en poids d'un copolymère éthylène-butylène commercialisé sous la référence GI 3000 (de masse molaire moyenne en poids égale à 3000) par la société NISSO CHEMICAL,
b) 21,3% en poids d'isophoronediisocyanate (noté IPDI).

Le mode opératoire est le même que celui décrit dans l'exemple 1, sauf le solvant utilisé pour la synthèse et la mise en dispersion qui est de la méthyl éthyl cétone. La neutralisation des fonctions amines tertiaires n'est effectuée qu'à 75%, et non 100% comme dans l'exemple 1.

On obtient un polyuréthane cationique ou cationisable à caractère élastique en dispersion dans l'eau, présentant les caractéristiques suivantes :

Taille de particule : 700 nm
Extrait sec : 10%
pH = 5
Viscosité : entre 5 et 20 cps

Recouvrance élastique instantanée après 100% de déformation : 70%

**Exemple 3 :** Gel de coiffage comprenant un polyuréthane selon l'invention

On formule une composition de gel de coiffage comprenant le polyuréthane selon l'invention de l'exemple 1.

La composition présente la formulation suivante (en poids par rapport au poids total de la composition) :

| | |
|---|---|
| Polyuréthane selon l'invention | 4% |
| Jaguar HP 105 | 4% |
| (hydroxypropyl guar, commercialisé par la société RHODIA) | |
| Eau | 4% |

Appliqué sur les cheveux, ce gel leur assure un coiffage facile et souple.

**Exemple 4 :** Mousse de coiffage comprenant un polyuréthane selon l'invention

On formule une composition de coiffage comprenant le polyuréthane selon l'invention de l'exemple 1.

La composition présente la formulation suivante (en poids par rapport au poids total de la composition) :

| | |
|---|---|
| Polyuréthane selon l'invention | 2% |
| Monolaurate de sorbitane oxyéthyléné (20 OE) | 0,2% |
| Eau | 92,8% |
| Isobutane/propane/butane (50/25/25) | 5% |

Appliqué sur les cheveux à l'aide d'un aérosol, cette mousse assure aux cheveux un coiffage facile et souple.

## Revendications

1. Polyuréthane cationique ou cationisable à caractère élastique **caractérisé en ce qu'**il est formé de la réaction :

    (a1) d' au moins un motif cationique ou cationisable résultant d'au moins une amine tertiaire ou quaternaire présentant au moins deux fonctions réactives à H labiles,
    (a2) d'au moins un mélange d'au moins deux motifs non ioniques différents présentant au moins deux fonctions réactives à H labiles,
    (b) d'au moins un composé comportant au moins deux fonctions isocyanate,

    le mélange de motifs non ioniques (a2) étant constitué d'un mélange d'au moins un premier polymère non ionique choisi parmi les homopolymères et copolymères d'oléfines, et d'au moins un second polymère non ionique différent d'un copolymère d'oléfine et choisi parmi les homopolymères et copolymères non ioniques, le ou lesdits premiers polymères non ioniques et le ou lesdits seconds polymères non ioniques portant à leurs extrémités des fonctions réactives à hydrogène labile et ayant une température de transition vitreuse (Tg), mesurée par analyse enthalpique différentielle, inférieure à 10 °C.

2. Polyuréthane cationique ou cationisable à caractère élastique selon la revendication 1, **caractérisé en ce que** le

ou lesdits premiers polymères non ioniques et le ou lesdits seconds polymères non ioniques formant les motifs non ioniques (a2) présentent une température de transition vitreuse, mesurée par analyse enthalpique différentielle, inférieure à 0 °C, de préférence inférieure à -10 °C.

**3.** Polyuréthane cationique ou cationisable à caractère élastique selon la revendication 1 ou 2 **caractérisé en ce que** les homopolymères et copolymères d'oléfines sont des homopolymères et copolymères présentant des motifs choisi parmi les motifs d'éthylène, de propylène, de 1-butylène, de 2-butylène, d'isobutylène, de 1,2-butadiène, de 1,4-butadiène et d'isoprène.

**4.** Polyuréthane cationique ou cationisable à caractère élastique selon la revendication 3 **caractérisé en ce que** le premier polymère non ionique est un copolymère d'éthylène et de butylène.

**5.** Polyuréthane cationique ou cationisable à caractère élastique selon l'une quelconque des revendications précédentes, **caractérisé en ce que** les homopolymères et/ou copolymères non oléfines formant les motifs non ioniques (a2) sont choisis parmi les poly(méth)acrylates, les polyamides, les polyéthers hydrocarbonés, fluorés ou perfluorés, les polyesters, les polyalkylsiloxanes, et les polycarbonates hydrogénés, fluorés ou perfluorés.

**6.** Polyuréthane cationique ou cationisable à caractère élastique selon la revendication 5 **caractérisé en ce que** les polyéthers sont choisis parmi les poly(oxyde d'alkylène en $C_2$-$C_4$) de masse moléculaire en nombre comprise entre 400 et 50000, de préférence le poly(oxyde de tétraméthylène).

**7.** Polyuréthane cationique ou cationisable à caractère élastique selon l'une quelconque des revendications précédentes **caractérisé en ce que** les motifs non ioniques (a2) résultent de la réaction du mélange de poly(oxyde de tétraméthylène) et de copolymère présentant des motifs d'éthylène, de 1,2-butadiène et/ou de 1,4-butadiène.

**8.** Polyuréthane cationique ou cationisable à caractère élastique selon l'une quelconque des revendications précédentes **caractérisé en ce que** le ou les premiers polymères d'oléfines et le ou les seconds polymères non oléfines formant les motifs non ioniques (a2) ont une masse moléculaire en nombre comprise entre 400 et 50000, de préférence entre 400 et 30000, mieux entre 400 et 25000.

**9.** Polyuréthane cationique ou cationisable à caractère élastique selon l'une quelconque des revendications précédentes **caractérisé en ce que** les motifs non ioniques (a2) représentent de 10 à 99,9%, de préférence de 20 à 99%, mieux de 30 à 85%, en poids du polyuréthane cationique ou cationisable.

**10.** Polyuréthane cationique ou cationisable à caractère élastique selon l'une quelconque des revendications précédentes, **caractérisé en ce que** les motifs cationiques ou cationisables (a1) résultent d'au moins une amine tertiaire ou quaternaire présentant deux fonctions réactives à hydrogène labile.

**11.** Polyuréthane cationique ou cationisable à caractère élastique selon la revendication 10, **caractérisé en ce que** les motifs cationiques ou cationisables (a1) résultent d'une amine choisie parmi les amines de formules suivantes :

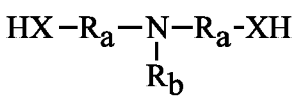

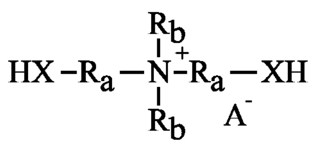

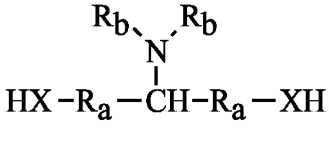

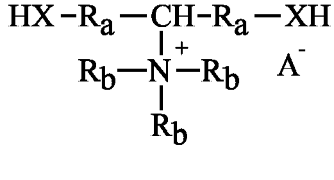

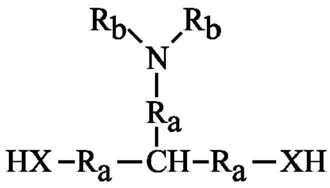

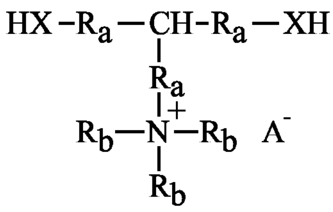

chaque $R_a$ représente indépendamment un groupe alkylène en $C_1$-$C_6$, linéaire ou ramifié, cycloalkylène en $C_3$-$C_6$ ou arylène, ou leurs mélanges ; tous pouvant être substitués par un ou plusieurs atomes d'halogène et comporter un ou plusieurs hétéroatomes choisis parmi O, N, PetS,
chaque $R_b$ représente indépendamment un groupe alkyle en $C_1$-$C_6$, cycloalkyle en $C_3$-$C_6$ ou aryle, ou leurs mélanges ; tous pouvant être substitués par un ou plusieurs atomes d'halogène et comporter un ou plusieurs atomes d'halogène et comporter un ou plusieurs hétéroatomes choisis parmi O, N, P et S,
chaque X représente indépendamment un atome d'oxygène ou de soufre ou un groupe NH ou $NR_c$, où $R_c$ représente un groupe alkyle en $C_1$-$C_6$, et
$A^-$ représente un contre-ion physiologiquement acceptable.

**12.** Polyuréthane cationique ou cationisable à caractère élastique selon la revendication 11, **caractérisé en ce que** les motifs cationiques ou cationisables (a1) résultent de la réaction de la N-méthyldiéthanolamine ou de la N-tert-butyldiéthanolamine.

**13.** Polyuréthane cationique ou cationisable à caractère élastique selon la revendication 10, **caractérisé en ce que** les motifs (a1) résultent de la réaction d'au moins un polymère à fonction(s) amine tertiaire et/ou quaternaire, portant à leurs extrémités des fonctions réactives à hydrogène labile choisies parmi -OH, $-NH_2$, $-NHR_c$ ou -SH, et ayant une masse moléculaire en poids comprise entre 400 et 10 000, $R_c$ représentant un groupe alkyle en $C_1$-$C_6$.

**14.** Polyuréthane cationique ou cationisable à caractère élastique selon l'une quelconque des revendications précédentes **caractérisé en ce que** les motifs cationiques ou cationisables (a1) représentent de 0,1 à 90%, de préférence de 1 à 30%, mieux de 1 à 20%, en poids du polyuréthane cationique ou cationisable.

**15.** Polyuréthane cationique ou cationisable à caractère élastique selon l'une quelconque des revendications précédentes, **caractérisé en ce que** le ou les composés (b) comportant au moins deux fonctions isocyanate sont choisis parmi les diisocyanates ou les mélanges d'un diisocyanate et d'un isocyanate comportant plus de deux fonctions isocyanates, ledit isocyanate représentant de 0,1 à 10% en poids du poids dudit mélange.

**16.** Polyuréthane cationique ou cationisable à caractère élastique selon la revendication 15, **caractérisé en ce que** les diisocyanates choisis parmi le méthylènediphényldiisocyanate, le méthylènecyclohexanediisocyanate, l'isophoronediisocyanate, le toluèn-diisocyanate, le naphtalènedüsocyanate, le 1,4-butanediisocyanate et l'1,6-hexanediisocyanate.

**17.** Polyuréthane cationique ou cationisable à caractère élastique selon l'une quelconque des revendications précédentes **caractérisé en ce qu'**il contient éventuellement au moins un motif non ionique (a3) contenant au moins deux fonctions à hydrogène labile capables de réagir avec le ou lesdits composés (b) comportant deux fonctions isocyanate.

**18.** Polyuréthane cationique ou cationisable à caractère élastique selon la revendication 17 **caractérisé en ce que** le ou les composés non ioniques monomères formant les motifs non ioniques (a3) sont choisis parmi les diols en $C_1$-$C_{12}$, de préférence le néopentylglycol, l'hexa(éthylèneglycol), le 1,2-éthanediol, le 1,2-propanediol et le 1,3-propanediol, et les aminoalcools en $C_1$-$C_6$, de préférence l'aminoéthanol.

**19.** Polyuréthane cationique ou cationisable à caractère élastique selon la revendication 17 ou 18, **caractérisé en ce que** les motifs non ioniques (a3) représentent de 0 à 50%, de préférence de 0 à 40%, mieux de 0 à 30%, en poids du polyuréthane cationique ou cationisable.

**20.** Polyuréthane cationique ou cationisable à caractère élastique selon l'une quelconque des revendications précédentes, **caractérisé en ce qu'**il présente au moins deux températures de transitions vitreuses (Tg) différentes, au moins une de ces Tg étant inférieure à 10 °C et au moins une autre étant supérieure ou égale à 20 °C.

**21.** Polyuréthane cationique ou cationisable à caractère élastique selon l'une quelconque des revendications précédentes, **caractérisé en ce qu'**il présente une recouvrance instantanée comprise entre 5 et 95 %, de préférence entre 20 et 90 % et en particulier entre 35 et 85 %.

**22.** Polyuréthane cationique ou cationisable à caractère élastique selon l'une quelconque des revendications précédentes, **caractérisé en ce qu'**il présente une viscosité comprise entre 1 et 100 cps.

**23.** Composition cosmétique contenant au moins un polyuréthane cationique ou cationisable à caractère élastique selon l'une quelconque des revendications 1 à 22.

**24.** Composition cosmétique selon la revendication 23, **caractérisée en ce qu'**il s'agit d'une laque pour cheveux et qu'elle contient de 0,5 à 15 % en poids de polyuréthane cationique ou cationisable selon l'une quelconque des revendications 1 à 22.

**25.** Composition cosmétique selon la revendication 23, **caractérisée en ce qu'**il s'agit d'un vernis à ongles et qu'elle contient de 0,5 à 40 % en poids de polyuréthane cationique ou cationisable selon l'une quelconque des revendications 1 à 22.

**26.** Composition cosmétique selon la revendication 23, **caractérisée en ce qu'**il s'agit d'une composition de maquillage de la peau, des lèvres et des phanères et qu'elle contient de 0,5 à 20 % en poids de polyuréthane cationique ou cationisable selon l'une quelconque des revendications 1 à 22.

**27.** Utilisation d'un polyuréthane cationique ou cationisable à caractère élastique selon l'une quelconque des revendications 1 à 22 dans une laque de cheveux.

**28.** Utilisation d'un polyuréthane cationique ou cationisable à caractère élastique selon l'une des revendications 1 à 22 pour former un film protecteur sur les ongles.

**29.** Utilisation d'un polyuréthane cationique ou cationisable à caractère élastique selon l'une des revendications 1 à 22 dans une composition de maquillage de la peau, des lèvres et des phanères.

**30.** Utilisation d'un polyuréthane cationique ou cationisable à caractère élastique selon l'une quelconque des revendications 1 à 22 pour la mise en forme permanente des cheveux.

**31.** Utilisation d'un polyuréthane cationique ou cationisable à caractère élastique selon l'une quelconque des revendications 1 à 22 pour la formulation de laques, de mousses ou de gels de coiffage.

**32.** Utilisation d'un polyuréthane cationique ou cationisable à caractère élastique selon l'une des revendications 1 à 22 pour une application rincée sur les cheveux.

Office européen des brevets

# RAPPORT DE RECHERCHE EUROPEENNE

Numéro de la demande
EP 05 30 0762

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 02/09655 A (L'OREAL) 7 février 2002 (2002-02-07) * page 1, ligne 6-13 * * page 2, ligne 10 - page 4, ligne 1 * * page 6, ligne 1 - page 10, ligne 18 * * page 12, ligne 4 - page 14, ligne 6 * * page 15, ligne 9-21 * * revendications; exemples * ----- | 1-32 | C08G18/08 A61K7/11 A61K7/02 |
| X | WO 02/10243 A (3M INNOVATIVE PROPERTIES COMPANY) 7 février 2002 (2002-02-07) * page 3, ligne 31 - page 4, ligne 9 * * page 6, ligne 19 - page 12, ligne 28 * * page 13, ligne 24 - page 15, ligne 9; revendications 1-9,11-26; exemples 56-62 * ----- | 1-32 | |
| X | WO 02/10242 A (3M INNOVATIVE PROPERTIES COMPANY) 7 février 2002 (2002-02-07) * page 1, ligne 5-7 * * page 5, ligne 30 - page 12, ligne 27 * * page 13, ligne 18 - page 17, ligne 26 * * page 22, ligne 19 - page 23, ligne 21; revendications 1-9,11-28 * ----- | 1-32 | DOMAINES TECHNIQUES RECHERCHES (IPC) |
| X | WO 02/36653 A (CROMPTON CORPORATION) 10 mai 2002 (2002-05-10) * page 1, ligne 8-11 * * page 3, ligne 6 - page 7, ligne 8; revendications; exemples * ----- | 1-3,5-22 | C08G A61K |
| A | WO 02/32978 A (L'OREAL; MOUGIN, NATHALIE) 25 avril 2002 (2002-04-25) * page 1, ligne 3-5 * * page 1, ligne 31 - page 10, ligne 12; revendications 9,10 * ----- -/-- | 1-32 | |

Le présent rapport a été établi pour toutes les revendications

| Lieu de la recherche | Date d'achèvement de la recherche | Examinateur |
|---|---|---|
| Munich | 11 novembre 2005 | Otegui Rebollo, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................................................
& : membre de la même famille, document correspondant

2

EPO FORM 1503 03.82 (P04C02)

Office européen des brevets

# RAPPORT DE RECHERCHE EUROPEENNE

Numéro de la demande
EP 05 30 0762

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| P,X | EP 1 543 819 A (L'OREAL) 22 juin 2005 (2005-06-22) * alinéas [0001], [0009] - [0049]; revendications 11-29 * ----- | 1-32 | |
| | | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 11 novembre 2005 | Otegui Rebollo, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................
& : membre de la même famille, document correspondant

2

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 30 0762

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-11-2005

| Document brevet cité au rapport de recherche | Date de publication | Membre(s) de la famille de brevet(s) | Date de publication |
|---|---|---|---|
| WO 0209655 A | 07-02-2002 | AU 8238701 A | 13-02-2002 |
| | | EP 1307177 A2 | 07-05-2003 |
| | | JP 2004515466 T | 27-05-2004 |
| | | US 2004141942 A1 | 22-07-2004 |
| | | US 6613314 B1 | 02-09-2003 |
| WO 0210243 A | 07-02-2002 | AT 302802 T | 15-09-2005 |
| | | AU 7294601 A | 13-02-2002 |
| | | BR 0112733 A | 24-06-2003 |
| | | CA 2415854 A1 | 07-02-2002 |
| | | CN 1444612 A | 24-09-2003 |
| | | DE 60112948 D1 | 29-09-2005 |
| | | EP 1305351 A1 | 02-05-2003 |
| | | JP 2004505137 T | 19-02-2004 |
| | | MX PA03000453 A | 05-04-2004 |
| | | US 6433073 B1 | 13-08-2002 |
| WO 0210242 A | 07-02-2002 | AU 1946001 A | 13-02-2002 |
| | | EP 1309640 A1 | 14-05-2003 |
| | | JP 2004505136 T | 19-02-2004 |
| | | US 6605666 B1 | 12-08-2003 |
| WO 0236653 A | 10-05-2002 | US 6339125 B1 | 15-01-2002 |
| WO 0232978 A | 25-04-2002 | AU 9569201 A | 29-04-2002 |
| | | EP 1326908 A1 | 16-07-2003 |
| | | FR 2815350 A1 | 19-04-2002 |
| | | JP 2004511637 T | 15-04-2004 |
| | | US 2004052753 A1 | 18-03-2004 |
| EP 1543819 A | 22-06-2005 | CA 2490699 A1 | 19-06-2005 |
| | | CN 1650840 A | 10-08-2005 |
| | | FR 2863884 A1 | 24-06-2005 |
| | | JP 2005220127 A | 18-08-2005 |

EPO FORM P0460

Pour tout renseignement concernant cette annexe : voir Journal Officiel de l'Office européen des brevets, No.12/82